# EUROPEAN PATENT SPECIFICATION

(11) **EP 1 830 751 B1**
(45) Date of publication and mention of the grant of the patent: **31.03.2010**
(21) Application number: 05850335.0
(22) Date of filing: 22.12.2005
(51) Int. Cl.: A61F 2/30

(54) **A METHOD OF SURFACE FINISHING A BONE IMPLANT**
VERFAHREN ZUR OBERFLÄCHENBEARBEITUNG EINES KNOCHENIMPLANTATS
PROCEDE DE FINITION DE SURFACE D'UN IMPLANT OSSEUX

(30) Priority: 23.12.2004 EP 04030665
(43) Date of publication of application: 12.09.2007
(73) Proprietor: Smith & Nephew Orthopaedics AG, 6343 Rotkreuz (CH)
(72) Inventor: ZINGER, Olivier, CH-5032 Rohr AG (CH); SCHMOTZER, Hans, CH-8048 Zürich-Altstetten (CH)
(74) Representative: Popp, Eugen
(86) International application number: PCT/EP2005/013905
(87) International publication number: WO 2006/066936

(56) References cited:
- EP-A- 0 987 031
- WO-A-2004/008984
- US-A- 4 365 359
- US-A- 5 344 494
- US-A- 5 603 338
- US-A1- 2002 143 404
- US-A1- 2004 153 154

## Description

The present invention relates to a method of surface finishing a bone implant for particular use in orthopaedic or dental procedures.

Such an implant is preferably manufactured so that in use it becomes integrated into the bone tissue. The implant must also be made of a material that is non-corrosive and that does not produce any immunological reaction causing rejection by the body. Typically, therefore such implants are metallic in nature and usually made from titanium, zirconium, niobium, or tantalum, or an alloy based on any of the aforesaid elements; medical grade stainless steel, or a cobalt-chromium alloy could also be used.

Events that lead to the integration of an implant into bone, and hence that determine the long-term performance of the implant, take place largely at the interface formed between the tissue and the implant. The development of this interface is complex and is influenced by numerous factors, including surface chemistry, surface charge, surface topography and surface contamination of the implant. To improve bone tissue integration, various techniques have been used to increase the surface roughness of titanium implants, and include: machining/micromachining, particle blasting, titanium plasma-spraying, chemical/electrochemical etching, particle blasting and chemical etching, electrochemical anodization, and pulsed laser ablation. Among these methods, abrasive blasting, also called sand blasting or grit blasting, is one of the most commonly found for metallic implants having surfaces that come into contact with bone that is to inter-grow therewith. Implants of this kind are used as prostheses in orthopaedics, for replacing broken or diseased bone, and in dentistry, for building artificial teeth. Nowadays, abrasive blasting is widely used to produce micro-rough surfaces on implantable devices, with typically 4-6 micrometers of Ra (average roughness, according to ISO 4287-1997 and ASME B46.1-1995). This process is widely used because it is efficient for roughening a surface while being cost effective and shows excellent clinical results. In a histological study of well-fixed grit-blasted Ti-6Al-7Nb stems of hip replacements, [A. Zweymüller, F. K. Lintner and M. F. Semlitsch, Journal of Clinical Orthopedics 235, 195-206 (1988*)*], it was found that excellent osseo-integration had been obtained as a result of the micro-roughness of the implant surfaces. It has also been found that osseo-integration occurs even under osteoporotic conditions: a grit-blasted Ti-6Al-7Nb prosthesis retrieved from a 100-year-old patient 3 years after arthroplasty showed sound bony fixation [D. K. Lester and P. Campbell, American Journal of Orthopaedics 25(1), 30-34 (1996*)*]. It has also been reported that an examination of 17 acetabular components that had been *in situ* for between 10 days and 2.5 months found that, during the insertion of the treated socket, the rough cp titanium surface becomes covered with a slurry of bone particles and blood plasma. This mixture of living cells, inorganic substances, and native bone-growth promoting agents (BMPs, growth factors, etc.) adheres to the surface and allows osteogenesis to occur, regardless of whether or not the implant is in direct contact with the host bone, [F. K. Lintner, M. Huber, G. Böhm, J. Attems and R. Wais, in 15 Jahre Zweymüller-Hüftendoprothese, III. Wiener Symposium, K. Zweymüller, Eds. (Verlag Hans Huber, Bern, 1996) pp. 131-137*].* It has been reported by Delaunay & Kapandji [C. P. Delaunay and A. I. Kapandji, Journal of Arthroplasty 11(6), 643-652 (1996*)*] that they obtained a cumulative 6-7 year survival rate of approximately 98% when using a "Zweymüller stem". Extended clinical follow-up by these authors also found that grit-blasted cp titanium threaded cups to have a 9-10 year survival rate of approximately 99% [C. P. Delaunay and A. I. Kapandji, Journal of Clinical Orthopaedics 340, 130-141 (1997*)*; and C. P. Delaunay and A. I. Kapandji, Acta Orthop Scand 69(4), 379-383 (1998*)*].

US Patent 5,456,723 discloses a method of producing such a metallic bone implant with a micro-roughness of 2µm or less by subjecting the surface of the implant to pickling in a reducing acid. Such a micro-roughness may be applied directly to the surface of the implant by the pickling process or be super-imposed upon a micro-roughness having an Rt (peak to valley height, according to ISO 4287-1997 and ASME B46.1-1995) in excess of 10µm produced by sand-blasting. In the latter case, the pickling step is so long and aggressive that the amount of the base material removed enables direct release and detachment of all the particles left behind by the sand-blasting, while producing an additional porous microtopography smaller than 2µm.

Many studies have analyzed the wear particles generated in joint replacement articulations and their effects on periprosthetic tissues. Additional third-body wear has been reported in cobalt-based alloy bearing surfaces for total hip replacement (THR) endoprotheses, originating for example from radio-pacifying agents in bone cements and from hydroxyapatite coatings [M. Rokkum, M. Brandt, K. Bye et al., *The Journal of Bone and Joint Surgery 81-B(4), 582-589 (1999)*]. Extensive surface inclusions from silicon- and/or aluminium-containing materials were found when five retrieved cementless implants with grit blasted surfaces were analyzed [J. L. Ricci, F. J. Kummer, H. Alexander and R. S. Casar, Journal of Applied Biomaterials 3, 225-230 (1992*)*]. Evidence of the same contaminants was found in soft-tissue samples adjacent to these implants, and also in a variety of new, non-implanted devices from different manufacturers. While in one experimental study alumina-blasting particles were considered to be a possible cause of tissue breakdown [B. W. Darvell, N. Samman, W. K. Luk, R.K.F. Clark and H. Tideman, Journal of Dentistry 23(5), 319-322 (1995*)*], in other experimental studies these surface contaminants on blasted titanium implants seemed not to have any negative effects on the rate of bone on-growth [V. M. Goldberg, S. Stevenson, J. Feighan and D. Davy, Clinical Orthopaedics 319, 122-129 (1995*)*; and A. Piattelli, L. Manzon, A. Scarano, M. Paolantonio and M. Piattelli, International Journal of Oral & Maxillofacial Implants 13, 805-810 (1998*)*]. More recently, Plenk et al. [H. Plenk Jr, M. Boehler, I. Steffan and A. Walter, European Cells and Materials 7(1), 78 (2004*)*]] showed that particle impaction of blasted implant surfaces leads to contamination of peri-implant tissues and metallic wear, but seems not directly to hinder bony anchorage. However, alumina particles produce increased third-body wear on both, the low and high carbon cobalt-based alloys studied. As a consequence, cobalt-based alloy wear particles lead to a pronounced foreign body reaction with all signs of an immune response. This work completes a previous study describing such adverse tissue reaction to metallic wear particles [M. Boehler, F. Kanz, B. Schwartz, I. Steffan, A. Walter, H. Plenk Jr and K. Knahr, The Journal of Bone and Joint Surgery 84-B, 128-136 (2002*)*]*.*

It will thus be appreciated that there is a need to develop a method which can reduce or substantially eliminate the hard particles contaminating grit-blasted surfaces without affecting too much the overall topography because a combination of topographies in the micrometre and nanometre range has shown to influence significantly the biological performance of implantable titanium devices in orthopaedic medicine and in dentistry.

One such approach for achieving this, is disclosed in US2004/0016651, wherein the use of blasting particles, in particular iron, is described for roughening the surface of the implant. Once the surface has been blasted and patterned, the blasting particles are selectively etched from the implant, in a stripping step. The etch used to remove these contaminants, is selective to the material of the blasting particles, wherein a stripping time of at least 60 minutes, preferably, 90 minutes, has to be observed to achieve an implant surface which is substantially free of residual iron.

Further relevent prior art is discussed below:

WO 2004/008984 relates to a method for treating an implant surface intended for implantation into bone tissue, wherein a micro-roughness comprising pores and peaks having a pore diameter of ≤ 1 µm, a pore depth of ≤ 500 nm, and a peak width, at half the pore depth, of from 15 to 150 % of the pore diameter is provided.

In US 4,365,359 a PMMA film is fixedly adhered to a prosthetic element by applying PMMA to the surface of the prosthetic element in the presence of a silane coupling agent. The resultant prosthesis is adapted to be joined to bone by means of bone cement.

US 5,603,338 discusses a method in which the surface of a device that is surgically implantable in living bone is prepared. The device is made of titanium with a native oxide layer on the surface. The method of preparation comprises the steps of removing the native oxide layer from the surface of the device and performing further treatment of the surface substantially in the absence of unreacted oxygen.

US 2002/0143404 describes an implant which includes a main body member having biocompatibility, and particles formed of bioactive material and dispersedly provided at the surface of an embedded section of the main body member. Each of the particles has a part embedded in the embedded portion and the other part protruding from the embedded portion. The main body member is formed of titanium or titanium alloy. The particles having osteo-conduction are formed of a material selected from among a group consisting of sintered substances of hydroxylapatite, alpha-tricalcium phosphate, beta-tricalcium phosphate, tetra-calcium phosphate, a single substance of amorphous calcium phosphate, monetite, brushite, 45S4 glass, and a mixture of them. It is desirable that the embedded section surface has a surface roughness in a range of 5 to 50 µm.

US 5,344,494 discloses a method for the cleaning of roughened surface or porous-coated medical implants of all kinds. In the cleaning method, the roughened surface or porous-coated implant is subjected to blasting with particulate solids selected from the solids that are soluble in a biocompatible non-toxic solvent and solids that deliquesce, sublimate or vaporize. The particulate solids must be sufficiently hard to provide effective impact for dislodging debris and loosely attached particles from implant surfaces. After blasting, the surface is optionally treated with ultrasonic vibration to further remove debris particulates.

The impacted surfaces may also be rinsed with a biocompatible liquid. After cleaning, the surfaces may be passivated by immersion in conventional passivation solutions or a solution of non-aggressive oxyanions.

The object of the present invention is to provide a method of surface finishing a bone implant that produces a rough surface, whilst reducing contamination caused by blasting media. This is solved by the method of surface finishing a bone implant in accordance with independent claim 1. Further preferred embodiments are given in the dependent claims.

The claimed invention can be better understood in view of the examples of a base station described hereinafter. In general, the described examples describe preferred embodiments of the invention. The attentive reader will note, however, that some aspects of the described examples extend beyond the scope of the claims. To the respect that the described examples indeed extend beyond the scope of the claims, the described examples are to be considered supplementary background information only.

According to a first aspect of the present invention there is provided a method of surface finishing a bone implant comprising the steps of roughening a surface of the implant by blasting with abrasive particles; pickling the surface-roughened implant in a pickling or etching solution; wherein the pickling step loosens any partially embedded abrasive blasting particles that may be contaminating the surface of the implant, by performing a short etch of the surface of the implant, in such a manner that the surface roughness remains substantially the same as before the pickling step, and in that the method comprises the additional step of cleaning the roughened surface of the implant by mechanical action to detach the loosened blasting particles there from. The term short etch means an etch of substantially less than 60-90 minutes, preferably of only some seconds, certainly a maximum of about 1-2 minutes.

This combination of steps fulfils the object of the invention. The pickling of the implant in the pickling solution loosens any partially embedded abrasive blasting particles that may be contaminating the surface of the implant. Those loosened abrasive particles as well as the firmly adhering abrasive blasting particles are then detached by the mechanical cleaning action. Hence, the pickling process should not be designed to clean the surface of the implant or to produce additional pitting of the surface of the implant, as in some prior art procedures, but only to unlock any partially embedded abrasive blasting particles. Similarly, the mechanical action also should not be designed to provide any additional roughening of the surface, all of which is carried out during the initial abrasive blasting, but only to remove the loosened and firmly adhering abrasive particles.

Preferably, the abrasive particles used to roughen the surface of the implant are ceramic and/or metal particles. If ceramic particles are used, these preferably comprise at least one of oxide particles, nitride particles and carbide particles.

Preferably also, the abrasive particles are propelled in a gaseous or liquid blasting medium.

Preferably also, the step of roughening the surface of the implant produces a surface roughness ranging from 3 to 7µm of Ra inclusive and from 20 to 70µm of Rt inclusive.

Preferably also, the pickling solution comprises one or a mixture of: a mixture of ammonium bi-fluoride and nitric acid; ammonium fluoride - ammonium bi-fluoride in acid mixtures; hydrofluoric acid based mixtures; sodium fluoride in acid mixtures; ammonium bi-fluoride and ammonium acetate in water; hydrochloric acid based mixtures; mixture of sulphuric and hydrochloric acid; and at least one fluoride salt, at least one acid and water.

Advantageously, the step of pickling the surface-roughened implant takes place by immersion of the implant in a mixture of ammonium bi-fluoride ([NH4)]HF2, 50g of powder for 1 litre), nitric acid (65% HNO3, 400 ml for 1 litre), and water to make up the solution to 1 litre. Preferably, the pickling takes place at room temperature, preferably a temperature of between 20 °C and 25°C, in particular at 22°C ± 2°C, for between 15 and 30 seconds.

Preferably also, the mechanical action comprises with the use of ultrasounds when the implant is immersed in a liquid medium after pickling. Alternatively, the mechanical action comprises the blasting of the implant with substantially non-abrasive or only slightly-abrasive particulate media. In this latter case, preferably the particulate media comprise at least one of dry-ice pellets, crystalline particles of water soluble material, and particles of bioactive blasting material. The blasting medium can be gaseous, for example filtered air or nitrogen, or liquid, for example water.

In cases where dry-ice pellets are used these preferably comprise carbon dioxide snow flakes. Preferably, also, they have an average diameter of 3 mm and are propelled in compressed air at an average pressure of 11 bars. The rate of supply of dry-ice pellets into the compressed air is preferably substantially of the order of 100 kilograms per hour. The time for blasting the surface lies in the range 20 seconds up to 3 minutes.

In cases where crystalline particles of water soluble material are used, these preferably comprise particles of at least one of a sugar, sodium chloride, sodium sulfate and a mixture of any of the aforesaid materials. Alternatively, where particles of bioactive blasting material are used these preferably comprise particles of calcium phosphate and/or calcium carbonate.

Preferably also, the bone implant is comprised of one of titanium, zirconium, niobium, tantalum, an alloy based on any of the aforesaid elements, medical grade stainless steel, and a cobalt-chromium alloy.

The present invention will now be described by way of example with reference to the accompanying drawings, in which:
Figs. 1 to 3 are a set of x60 magnified optical images of the surface of a hip-joint implant after surface finishing respectively using three different surface finishing methods including in Fig. 3 a method in accordance with the present invention;
Fig. 4 is a graph illustration showing the percentage contamination of the surface of an implant by abrasive particles after surface finishing using five different surface finishing methods including the three methods illustrated in Figs. 1 to 3 as measured by image analysis on Back-Scattered-Electrons (BSE) micrographs and by optical micrographs;
Fig. 5 is an image produced by back scattering electron imaging (BSE imaging) at a magnification of x100 showing contamination of the surface of an implant by abrasive particles after grit blasting; and
Fig. 6 is a similar image to that shown in Fig. 5 but at a magnification of x200 and showing contamination of the surface of a similar implant by abrasive particles after surface finishing using a method in accordance with the present invention;
Figs. 7 and 8 are graphs showing the roughness parameters Ra and Rt respectively of the surface of an implant after different surface treatment methods including methods in accordance with the present invention .

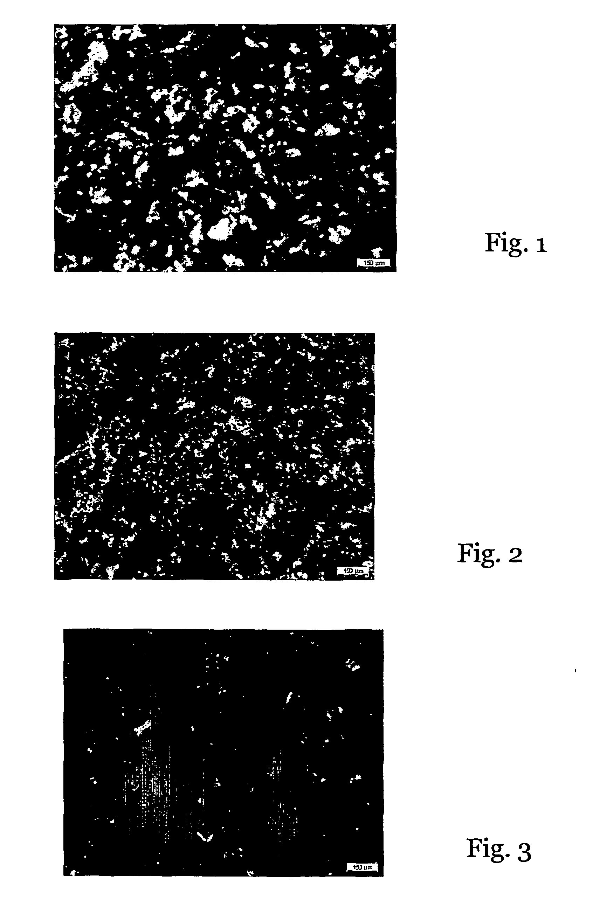

Figs. 1 to 3 respectively show the contamination of hip-joint implants by alumina particles after surface finishing using three different finishing methods. The hip-joint in each case was comprised of a Ti6Al7Nb alloy. In each image the contaminating alumina particles appear white or light grey in colour and the images were enhanced by a very short titanium etching applied for 20 seconds at 22 ± 2°C after each finishing treatment had been completed to obtain a better contrast with polarized light.

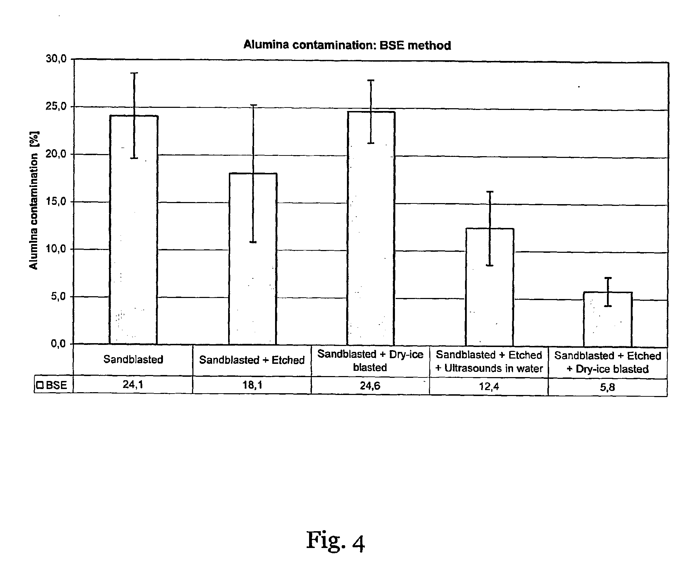

Fig. 4 shows graphically the mean percentage contamination of the surface of these implants obtained by image analysis on BSE micrographs and on optical micrographs after completion of five different surface finishing methods, including those used to produce Figs. 1 to 3.

To obtain the results from BSE micrographs the implant sample to be analyzed was introduced without any further treatment in a scanning electron microscope equipped with a BSE (Back-Scattered-Electron) detector. Five Back-Scattered-Electrons (BSE) micrographs were taken per sample at different randomly selected positions on the surface of the sample. The conditions used are the following: acceleration voltage: 20kV; spot-size: large (10); magnification: 100x; working distance: 25mm; detector adjustment: chemical contrast; amplification of the BSE signal: medium. Under such conditions, alumina appears black whereas the titanium surface appears white. An image analysis is then performed for each BSE micrograph with the contrast adjusted so that black corresponds to alumina. The sum of all the black areas compared to the entire surface of the micrograph analyzed gives the surface contamination in percent. The mean value as well as the standard deviation per sample are calculated from 5 different measurements performed on 5 different BSE micrographs. The accuracy of the method was controlled by Energy-Dispersive X-Ray (EDX) mapping performed in the same conditions (distance to detectors and magnification).

To obtain the results from optical micrographs the implant sample to be analyzed was chemically etched for 20 seconds at room temperature (22 ± 2°C) in a mixture of ammonium bifluoride ([NH4)]HF2, 50g of powder for 1 litre) and nitric acid (65% HNO3, 400 ml for 1 litre) completed with water to obtain 1 litre of solution. The sample was then carefully rinsed with osmotic or purified water and left to dry in air. This enables a satisfactory optical contrast to be obtained. Six pictures were taken randomly of the surface of the implant using a digital camera coupled to a microscope equipped with a polarizing filter. The conditions used were the following: magnification of 60x, polarizing filter adjusted to obtain the maximum dark-field. Under such conditions, alumina appears white whereas the titanium surface appears black. An image analysis was then performed for each optical micrograph with the contrast adjusted so that white corresponds to alumina. The sum of all the white areas compared to the entire surface of the micrograph analyzed gives the surface contamination in percent. The mean value as well as the standard deviation per sample were then calculated from the 6 different measurements performed on the 6 different optical micrographs.

Fig. 1 shows the contamination of the surface after a hip-joint has been grit blasted using alumina-sand particles. This is a conventional surface treatment for such implants and, as can be seen from the image and by reference to the block labelled 'Sandblasted' in Fig. 4, the surface contamination is relatively high. Fig. 2 shows the contamination of the surface of a similar hip-joint after another conventional surface treatment wherein the hip-joint is first grit blasted using alumina particles and then 'cleaned' by dry-ice blasting directly on the grit blasted surface. Fig. 2 shows that the contamination is only slightly reduced by the ice-blasting treatment. If reference is made to the block labelled 'Sandblasted + Dry-ice blasted' in Fig. 4. it can also be seen that the dry-ice blasting produces no improvement on the alumina contamination when measured by BSE.

Fig. 4 also shows the result, of etching the grit-blasted surface (see block labelled 'Sandblasted + Etched'). Again, there is no significant improvement in surface contamination.

In contrast, Fig. 3 shows the results of a treating the surface of a similar hip-joint using a method also in accordance with the present invention wherein after grit-blasting using alumina sand the hip-joint was subjected to a short pickling treatment of around 20 seconds by immersion in a pickling solution prior to being dry-ice blasted. The addition of the short pickling step produces a significant improvement on the subsequent cleaning step by dry-ice blasting. As shown in Fig. 4, the alumina contamination is 76% lower than the original grit-blasted surface on the mean values when measured with BSE (on the mean values). Measured with the optical method, the reduction of contamination is of the order of 96%

The difference in magnification, in surface sensitivity and in precision between the two methods explains this discrepancy. The BSE method is very accurate and can measure very fine particles but it also measures, as deep as 3-5 microns underneath the surface, the alumina particles that are completely embedded in the substrate. On the other hand, the optical method is only sensitive to large alumina particles which are very slightly embedded.

In addition, it can be seen from Fig. 4 that the use of ultrasounds in water after etching (Sandblasted + Etched + Ultrasounds in water) reduces the contamination by around 48% when measured with BSE (on the mean values). This is not as effective as using dry-ice blasting but is still significantly better than prior art procedures.

Grit blasting is a stochastic process leading to a rough topography. The fact is that the coarser contributions to roughness often hide the fine surface roughness features when considering the standard "integral" roughness parameters such as Ra or Rt, which are defined according to ISO 4287-1997 and ASME B46.1-1995 such that Ra is arithmetic average of the absolute values of all points of the profile and Rt is the maximum peak-to-valley height of the entire measurement trace. These "integral" roughness parameters are scale dependent and depend also on the cut-off wavelength applied when measuring same. Typically, the roughness parameter Ra obtained after alumina grit blasting lies between 3 to 7 micrometres. However, it is known that such blasted surfaces are characterized by numerous surface roughness features ranging from the 100 micrometres scale to the nanometre scale.

In the present method, a bone implant made by a conventional method from a biocompatible material, such as any of titanium, zirconium, niobium, tantalum, an alloy based on any of the aforesaid elements, medical grade stainless steel, and a cobalt-chromium alloy is surface roughened. The roughening is produced by blasting with abrasive particles to produce micro and sub-micro topography. The blasting particles are preferably ceramic particles, such as oxides (for example: alumina, zirconia, titania, fused titanium dioxide, fused aluminium oxide), nitrides (for example carbon nitride, silicon nitride or boron nitride) or carbides (for example chromium carbide, silicon carbide or boron carbide), or metal particles. The medium use to propel the blasting particles can be gaseous, such as air or nitrogen (that may or may not have been dried), or liquid, for example water. After this blasting, the surface contamination by the blasting material has been typically found to be between 15 and 40% when measured by BSE and 10 to 30% when measured optically. The Ra and Rt roughness parameters are typically 3≤ Ra ≤7µm and 20≤ Rt≤70µm respectively.

After surface blasting, the implant is subjected to a pickling process in a pickling or corrosive solution in order to loosen any partially embedded blasting particles from its surface. The exposure of the implant to the pickling solution must be carried out under controlled conditions for a controlled time in order to provide sufficient loosening of the partially-embedded blasting particles while minimizing topographical modifications to the surface of the implant. After pickling, the implant should be rinsed clean using osmotic or purified water and may be dried.

The pickling process loosens and unlocks the blasting particles from the surface of the implant, by performing a brief etch of the implant. Placement of the implant within the pickling (or etching) solution causes an etch, which is preferably isotropic, of the surface of the implant. It is clear, that this etch will also effect the regions around any embedded or partially embedded blasting particles. This etch thus causes the implant surface to loosen its hold on the blasting particles, and in places where the particles are only slightly embedded: will actually be etched away to completely free the blasting particle from the surface.

Preferably, the pickling or etching process is performed rapidly, with the corresponding solution attacking the surface of the implant in an appropriately aggressive manner to allow this. When the etch is performed quickly, the topography of the surface before and after the etch remains substantially unmodified. In other words, the surface roughness achieved by the blasting process, is substantially the same after the pickling step. Preferably, the substrate is etched by no more than 20µm; more preferably, the surface is etched by between 4µm and 10µm, and in further situations, the surface is etched by between 1µm and 2µm. It is, of course, possible for etches of greater than 20µm to be performed, and etches lower than 1µm to be performed, in order to remove blasting particles which are significantly deeply embedded, or in cases when the particles are very loosely bound or embedded.

There are significant advantages to be seen from etching the surface in this manner. As has been stressed, the etch is specifically tailored to rapidly etch the surface of the implant, and in this manner the roughness of the surface which is defined by the blasting step remains substantially unaltered. Certainly, from the point of view of a useful surface with regard to micrometre and nanometre patterning and subsequent tissue integration into the host body, this is the case. Additionally, as the etch is performed on the implant, rather than on the material making up the blasting particles, it allows for any of the above mentioned particles to be used as the blasting media. This is a significant advantage, as dependent upon the final requirements of the implant, different blasting media could be required or useful for giving different surface finishes. Furthermore, certain blasting materials are not compatible with living tissue, and therefore, any contamination of the implant with such materials could lead to complications within the patient after implant.

The pickling or etching solution may comprise any of the following compositions:
- a mixture of ammonium bi-fluoride and nitric acid;
- ammonium fluoride - ammonium bi-fluoride in acid mixtures (such as hydrochloric acid, or sulphuric acid, or nitric acid);
- hydrofluoric acid based mixtures;
- sodium fluoride in acid mixtures (such as hydrochloric acid, and/or nitric acid);
- ammonium bi-fluoride and ammonium acetate in water;
- hydrochloric acid based mixtures;
- mixture of sulphuric and hydrochloric acid;
- at least one fluoride salt, at least one acid and water.

In the last case, the fluoride salt is preferably selected from a group comprising ammonium fluoride, ammonium bi-fluoride, potassium fluoride or sodium fluoride, or mixture thereof, the concentration of the fluoride salt being between 0.1 to 6 wt. % of the pickling solution. The acid is preferably selected from a group comprising nitric acid, hydrochloric acid, sulphuric acid, phosphoric acid, acetic acid, lactic acid, oxalic acid, tartaric acid, and mixtures thereof, the concentration of the acid in the pickling solution being about 0.1 to about 6N. The pickling solution may additionally comprise a chemically inert, water-soluble salt, selected from a group comprising sodium chloride, sodium sulphate, sodium bisulphate, sodium phosphate, sodium hydrogen phosphate, sodium dihydrogen phosphate, sodium nitrate, potassium chloride, potassium sulphate, potassium bisulphate, potassium hydrogen phosphate, potassium dihydrogen phosphate, potassium nitrate, ammonium sulphate, and mixtures thereof, the concentration of the sulphate salt being about 0.5 to 8 wt. % of the pickling solution.

Various controlled conditions may be applied during the pickling process. The temperature of the pickling bath can be between 5°C and boiling point, which may be above 100°C. The pickling bath may also be agitated. The agitation may be achieved by mechanical means or by bubbling an inert gas through the pickling solution. The pickling bath may also be aerated or kept under inert atmosphere, for example an argon or nitrogen atmosphere. The immersion time of the implant can be from a few seconds to several minutes depending on the aggressivity or etching rate of the pickling solution. The immersion time must be adjusted in order to keep the topographical parameters of the roughened implant surface before etching substantially the same. Preferably, however, the etch should be performed rapidly, as this will leave the final surface after the etch with substantially the same surface roughness as was obtained with the blasting step. It has also been found that he efficacy of the pickling process can be enhanced electrochemically by anodic polarization of the implant in the bath.

After the pickling step, the implant undergoes a careful rinsing in water, preferably deionised and filtered water, in order to completely remove the pickling solution. Following this rinse, the method in accordance with the invention comprises a mechanical cleaning step in order to detach the blasting particles there from which have been loosened or loosened by the pickling process. Various forms of mechanical cleaning action including dry-ice blasting, as mentioned above, may be employed as follows:
- blasting of the surface of the implant using non- to slightly-abrasive particles at an appropriate pressure predetermined in order to avoid further roughening of the implant surface;
- ultrasonic cleaning of the implant in a liquid medium;
- liquid jetting of the implant;
- brushing the surface of the implant either manually or mechanically, for example with nylon brushes again in order to avoid further roughening of the implant surface.

Preferably, however, the surface of the implant is cleaned by blasting with a substantially non-abrasive particulate media such as one of the following:
- dry-ice pellets; for example made of compressed carbon dioxide snow flakes which have been extruded through a die. An alternative is to let liquid carbon dioxide flow directly through a special two-component concentric nozzle, the liquid expands as it exits and becomes a mixture of CO₂ snow (ice crystals) and gas which forms the core jet. In addition, compressed air (blasting medium) is fed in a ring shape;
- crystalline particles of water soluble material, such as particles of sugar(s), sodium chloride, sodium sulphate and the like, which can be easily removed from the implant surface, with or without the addition of flow/anti-caking agent(s);
- particles of bioactive blasting material, such as calcium phosphate or calcium carbonate, that are bioresorbable and to some extent water soluble but do not necessarily need to be removed from the surface.
The blasting medium can be gaseous, such as air, nitrogen or the like, which may be dried, or be liquid, such as water.

After the cleaning step, the surface contamination on the implant is further reduced but still present and is typically between 1 % and 10% when measured by BSE and between 0.1% and 5% when measured optically. The Ra and Rt roughness parameters are typically 3≤ Ra ≤7µm and 20≤ Rt ≤70µm respectively, which are the same as after the blasting to produce the roughened surface. It will thus be appreciated that the processing conditions used in the pickling step and the cleaning step are adjusted in order to keep the topographical parameters approximately the same.

With a conventional implant such as a hip-joint comprised of a Ti-6AL-7NB alloy in accordance with ISO 5832-11, it has been found that a surface treatment using the following method produces optimal results.

First, the surface of the implant is subjected to an abrasive blasting procedure using a conventional alumina particulate (Al₂O₃, Biloxit Type K20 or K24). Dependent on the blasting apparatus used the pressure applied for blasting can range between 3 and 8 bars inclusive. Such a procedure produces a surface roughness ranging from 4 to 6µm of Ra (average roughness, according to ISO 4287-1997 and ASME B46.1-1995). Next, the implant is pickled by immersion in a mixture of ammonium bi-fluoride ([NH4)]HF2, 50g of powder for 1 litre) and nitric acid (65% HNO3, 400 ml for 1 litre) in water. The pickling bath should be maintained in the temperature range 20°C to 25C, in particular at 22°C ± 2°C, i.e. approximately at room temperature. The immersion time of the implant in the bath should be for between 15 and 30 seconds. After this pickling step, the implant is carefully rinsed in water, preferably deionised and filtered water, in order to remove the pickling solution. Finally, the implant is subjected to dry-ice blasting wherein it is blasted directly using 3 mm (average diameter) CO₂ pellets at an average of 11 bars with compressed air, using a dry-ice pellet supply of 100 kilograms per hour. The duration of ice blasting is from between 20 seconds and up to 3 minutes, depending upon the previous parameters.

It has been found that the pickling step produces a maximum reduction of 40% of the alumina contamination (when considering mean values of contamination measured by the BSE method, and compared to a grit blasted surface). A mean reduction of 25% is measured on Ti-6Al-7Nb. The etching depth measured after 20 seconds of etching at 27°C on polished Ti-6Al-7Nb samples corresponds to 2.7µm for cp Ti (Grade 2) and to 1.4µm for Ti-6Al-4V (Grade 5, ELI) when measured with an optical profilometer FRT-MicroProf, equipped with a chromatic sensor CWL 0.3mm using the following method:
1. polishing the samples to a mirror finish, with a maximum Ra of 0.1 µm (finish N3 or lower);
2. protecting the surface (with a coating resistant to the acid mixture) in order to leave a free line approximately 2 mm in width;
3. immersing the sample in the pickling bath for the desired time, at the desired temperature to pickle the unprotected zone;
4. removing the coating;
5. measuring the depth of the etched groove with a Laser or an optical profilometer by
   - mapping 4 x 4 mm;
   - using a minimum point density of 80 pts/mm;
   - taking single profiles across the groove including both ridges in order to define the base line;
   - measuring the mean depth of the groove (on a minimum of 8 different profiles).

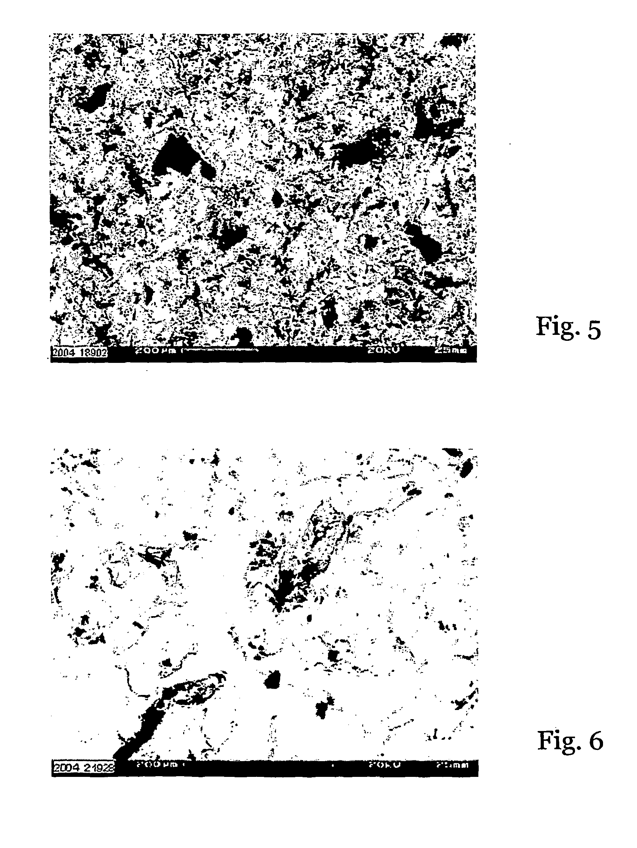

The improvement of the results over conventional surface treatment methods can be appreciated by a comparison of Figs. 5 and 6, which are both BSE images showing the contamination of the surface of an implant by abrasive particles. Fig. 5, which is at a magnification of x100, shows the surface contamination after a conventional grit blasting treatment using alumina particles whereas Fig. 6, which is a similar image but at a magnification of x200, shows the surface contamination after surface finishing using the aforesaid method in accordance with the present invention. In both cases the alumina particles appear black or dark grey and it is clear that the surface contamination in Fig. 6 where a method in accordance with the invention has been employed is significantly lower than that in Fig. 5.

As previously stated, the pickling process used in the method in accordance with the invention is not designed to clean or structure the surface of the implant significantly with regard to the values of the alumina contamination as well as the roughness parameters before and after the pickling process. Rather, the pickling process is designed to loosen any partially embedded blasting particles. This is achieved by a preferably isotropic and rapid etch of the implant itself, which leaves the resulting surface structure substantially the same as before the etch. This etch, however, results in the implant surface surrounding the embedded blasting particles being etched, and the grip/hold or physical bond between the blasting particle and implant being lessoned such that the particles are loosened, unlocked or even completely freed from the surface. Also, the mechanical cleaning step is not designed to induce any additional roughening of the treated surface with regard to the roughness parameters before and after the combined process but to detach the abrasive particles from the surface which have been loosened by the pickling process. Hence, the abrasive blasting procedure used initially should be designed to produce the degree of surface roughness required in accordance with the type of implant in question and its proposed use. This can be appreciated by a consideration of the roughness parameters shown in tabular form in the following Table 1. Here, the roughness parameters of the surface of an implant after different surface treatments are shown having been measured with a non-contact optical profilometer FRT-MicroProf, equipped with a chromatic sensor CWL 0.3mm (length of measurement = 5.6 mm; 1000 pts/mm; cut-off = 0.8 mm, 0.8 mm was ignored at the beginning and the end of the profile; the calculation was performed using a Gaussian filter and an attenuation factor of 50% at the cut-off wavelength). Mean values and standard deviations (STDEV) are calculated from 6 measurements. The

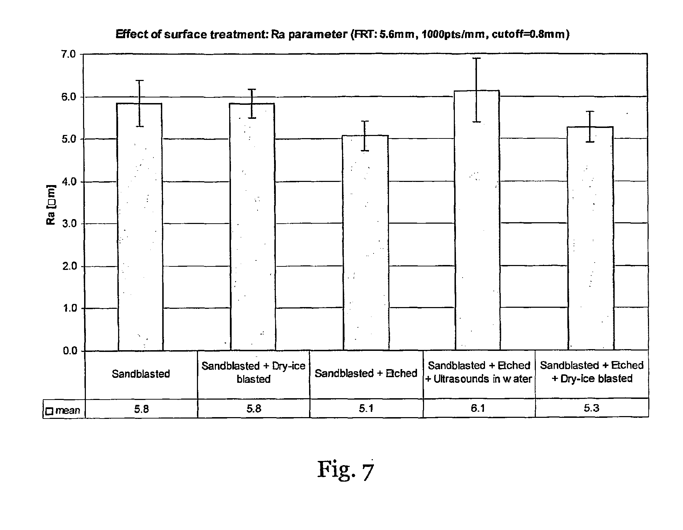

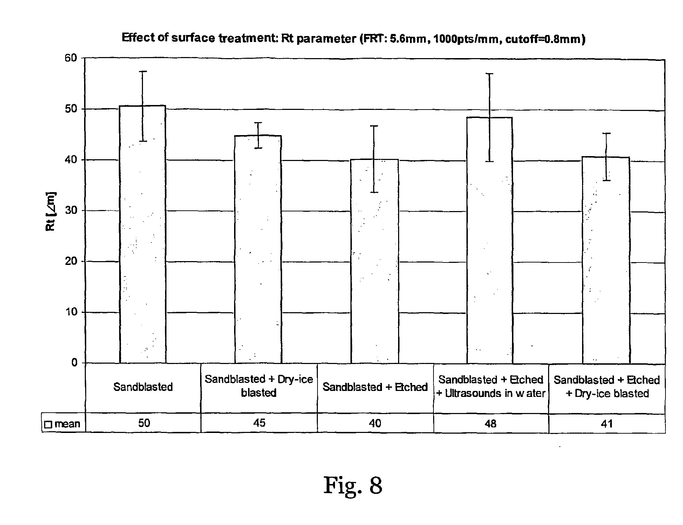

results for Ra and Rt are also shown graphically in Figs 7 and 8 respectively. These roughness parameters have stayed staying within the same range for the various surface finishing method used.

**Table 1**

| | Sandblasted | | Sandblasted + Dry-ice blasted | | Sandblasted + Etched | | Sandblasted + Etched + Ultrasounds in water | | Sandblasted + Etched + Dry-ice blasted | |
|---|---|---|---|---|---|---|---|---|---|---|
| | Mean | *STDEV* | Mean | *STDEV* | Mean | *STDEV* | Mean | *STDEV* | Mean | *STDEV* |
| Ra [µm] | 5.8 | *0.5* | 5.8 | *0.3* | 5.1 | *0.3* | 6.1 | *0.8* | 5.3 | *0.4* |
| Rq [µm] | 7.4 | *0.7* | 7.3 | *0.5* | 6.4 | *0.6* | 7.7 | *0.9* | 6.7 | *0.5* |
| Rt [µm] | 50.5 | *6.8* | 44.8 | *2.5* | 40.2 | *6.5* | 48.5 | *8.6* | 40.8 | *4.6* |
| Rz(DIN) [µm] | 37.1 | *2.8* | 35.9 | *1.9* | 30.7 | *2.7* | 38.5 | 4.5 | 31.6 | *2.8* |
| Rmax [µm] | 45.9 | *9.2* | 42.8 | *3.8* | 38.2 | *6.3* | 46.8 | 9.2 | 39.1 | *6.2* |
| Rsk[-] | 0.0 | *0.2* | -0.1 | *0.2* | 0.1 | *0.2* | 0.0 | *0.3* | -0.2 | *0.2* |

| | | | | | | | | | | |
|---|---|---|---|---|---|---|---|---|---|---|
| The different roughness parameters are defined according to ISO 4287-1997 and ASME B46.1-1995: Ra = arithmetic average of the absolute values of all points of the profile; Rq = root mean square (RMS) of the values of all points of the profile, Rt = maximum peak-to-valley height of the entire measurement trace, Rz(DIN) = arithmetic average of the maximum peak-to-valley height of the roughness values of 5 consecutive sampling sections over the filtered profile; Rmax = maximum individual roughness depth; Rsk = amplitude distribution skew | | | | | | | | | | |

## Claims

1. A method of surface finishing a bone implant comprising the steps of roughening a surface of the implant by blasting with abrasive particles;
pickling the surface-roughened implant in a pickling solution; and
cleaning the roughened surface of the implant by mechanical action to detach the loosened blasting particles therefrom,
wherein the pickling step comprises an etch of the implant surface which unlocks or loosens any partially embedded abrasive blasting particles that may be contaminating the surface of the implant,
**characterised in that**:
the pickling step is adjusted such that it leaves the surface of the implant with substantially the same roughness as is generated by the blasting with abrasive particles.

2. A method as claimed in Claim 1, wherein the etch is specific to the material of implant over the material comprising the blasting particles.

3. A method as claimed in either of Claims 1 or 2, wherein the etch is isotropic.

4. A method as claimed in any of Claims 1 to 3, wherein the surface of the implant is etched by less than 20µm by the pickling step.

5. A method as claimed in any of Claims 1 to 3, wherein the surface of the implant is etched by between 4µm and 10µm by the pickling step.

6. A method as claimed in any of Claims 1 to 3, wherein the surface of the implant is etched by between 2µm and 4µm by the pickling step.

7. A method as claimed in any of Claims 1 to 3, wherein the surface of the implant is etched by between 1µm and 2µm by the pickling step.

8. A method as claimed in any of Claims 1 to 7, **characterised in that** the abrasive particles used to roughen the surface of the implant are ceramic and/or metal particles.

9. A method as claimed in Claim 8, **characterised in that** the ceramic abrasive particles comprise at least one of oxide particles, nitride particles and carbide particles.

10. A method as claimed in any of Claims 1 to 9, **characterised in that** the abrasive particles are propelled in a gaseous or liquid blasting medium.

11. A method as claimed in any of Claims 1 to 10, **characterised in that** the step of roughening the surface of the implant produces a surface roughness ranging from 3 to 7µm of Ra inclusive.

12. A method as claimed in any of Claims 1 to 11, **characterised in that** the step of roughening the surface of the implant produces a surface roughness ranging from 20 to 70µm of Rt inclusive.

13. A method as claimed in any of Claims 1 to 12, **characterised in that** the pickling solution comprises one or a mixture of: a mixture of ammonium bi-fluoride and nitric acid; ammonium fluoride - ammonium bi-fluoride in acid mixtures; hydrofluoric acid based mixtures; sodium fluoride in acid mixtures; ammonium bi-fluoride and ammonium acetate in water; hydrochloric acid based mixtures; mixture of sulphuric and hydrochloric acid; and at least one fluoride salt, at least one acid and water.

14. A method as claimed in any of Claims 1 to 13, **characterised in that** the step of pickling the surface-roughened implant takes place by immersion of the implant in a mixtures of 50g of powder ammonium bi-fluoride, 400ml of 65% nitric acid and water to make up the solution to 1 litre.

15. A method as claimed in Claim 14, **characterised in that** the implant is immersed in the mixture of ammonium bi-fluoride, nitric acid and water at room temperature for between 15 to 30 seconds.

16. A method as claimed in Claim 14 or Claim 15, **characterised in that** the mixture of ammonium bi-fluoride, nitric acid and water is maintained at a temperature of between 20°C and 25°C, in particular 22 ± 2°C.

17. A method as claimed in any of Claims 1 to 16, **characterised in that** the mechanical action comprises the use of ultrasounds when located in a liquid medium.

18. A method as claimed in any of Claims 1 to 17, **characterised in that** the mechanical action comprises the blasting of the implant with a substantially non-abrasive particulate media.

19. A method as claimed in Claim 18, **characterised in that** the substantially non-abrasive particulate media are propelled in a gaseous or liquid blasting medium.

20. A method as claimed in Claim 18 or Claim 19, **characterised in that** the substantially non-abrasive particulate media comprise at least one of dry-ice pellets, crystalline particles of water soluble material, and particles of bioactive blasting material.

21. A method as claimed in Claim 20, **characterised in that** the dry-ice pellets comprise carbon dioxide snow flakes.

22. A method as claimed in Claim 20 or Claim 21, **characterised in that** the dry-ice pellets have an average diameter of 3 mm and are propelled in compressed air at an average pressure of 11 bars.

23. A method as claimed in Claim 22, **characterised in that** the rate of supply of dry-ice pellets into the compressed air is substantially of the order of 100 kilograms per hour.

24. A method as claimed in any of Claims 20 to 23, in which the blasting with dry ice pellets is for a duration of between 20 seconds up to 3 minutes.

25. A method as claimed in Claim 20, **characterised in that** the crystalline particles of water soluble material comprise particles of at least one of a sugar, sodium chloride, sodium sulphate and a mixture of any of the aforesaid materials.

26. A method as claimed in Claim 20, **characterised in that** the particles of bioactive blasting material comprise particles of calcium phosphate and/or calcium carbonate.

27. A method as claimed in any of Claims 1 to 26, **characterised in that** the bone implant is comprised of one of titanium, zirconium, niobium, tantalum, an alloy based on any of the aforesaid elements, medical grade stainless steel, and a cobalt-chromium alloy.

28. A method as claimed in any of Claims 1 to 27, wherein between the pickling step and the mechanical cleaning step, the implant is subjected to a rinse in water, in order to remove any pickling solution contaminants.

29. A method as claimed in Claim 28, wherein the water is deionised and filtered water.

## Patentansprüche

1. Verfahren zur Oberflächenbearbeitung eines Knochenimplantats, welches die Schritte eines Aufrauens einer Oberfläche des Implantats durch ein Bestrahlen mit Schleifpartikeln;
Abbeizen des Implantats mit der aufgerauter Oberfläche in einer Beizlösung; und
Reinigen der aufgerauten Oberfläche des Implantats durch eine mechanische Wirkung, um die losgelösten Strahlpartikel davon zu entfernen; enthält,
wobei der Beizschritt ein Ätzen der Implantatoberfläche enthält, welches jegliche teilweise eingebettete Strahlschleifpartikel loslöst oder ablöst, welche die Oberfläche des Implantats verunreinigen könnten,
**dadurch gekennzeichnet, dass**:
der Beizschritt derart eingestellt ist, dass er die Oberfläche des Implantats mit der im Wesentlichen gleichen Rauhigkeit, wie durch die Bestrahlung mit den Schleifpartikeln erzeugt, belässt.

2. Verfahren nach Anspruch 1, bei welchem das Ätzen spezifisch auf das Material des Implantats über dem Material ist, welches die Strahlpartikel enthält.

3. Verfahren nach Anspruch 1 oder 2, bei welchem das Ätzen isotropisch ist.

4. Verfahren nach einem der Ansprüche 1 bis 3, bei welchem die Oberfläche des Implantats durch den Beizschritt um weniger als 20 Mikrometer geätzt wird.

5. Verfahren nach einem der Ansprüche 1 bis 3, bei welchem die Oberfläche des Implantats durch den Beizschritt um weniger als 4 Mikrometer und 10 Mikrometer geätzt wird.

6. Verfahren nach einem der Ansprüche 1 bis 3, bei welchem die Oberfläche des Implantats durch den Beizschritt zwischen 2 Mikrometer und 4 Mikrometer geätzt wird.

7. Verfahren nach einem der Ansprüche 1 bis 3, bei welchem die Oberfläche des Implantats durch den Beizschritt zwischen 1 Mikrometer und 2 Mikrometer geätzt wird.

8. Verfahren nach einem der Ansprüche 1 bis 7, **dadurch gekennzeichnet, dass** die Schleifpartikel, welche dazu verwendet werden, um die Oberfläche des Implantats aufzurauen, Keramik- und/oder Metallpartikel enthalten.

9. Verfahren nach Anspruch 8, **dadurch gekennzeichnet, dass** die Keramik-Schleifpartikel zumindest eines aus Oxid-Partikeln, Nitrid-Partikeln und Karbid-Partikeln enthalten.

10. Verfahren nach einem der Ansprüche 1 bis 9, **dadurch gekennzeichnet, dass** die Schleifpartikel in einem gasförmigen oder flüssigen Strahlmedium angetrieben werden.

11. Verfahren nach einem der Ansprüche 1 bis 10, **dadurch gekennzeichnet, dass** der Schritt des Aufrauens der Oberfläche des Implantats eine Oberflächenrauhigkeit erzeugt, welche im Bereich zwischen 3 und 7 Mikrometer von Ra inklusive ist.

12. Verfahren nach einem der Ansprüche 1 bis 11, **dadurch gekennzeichnet, dass** der Schritt des Aufrauens der Oberfläche des Implantats eine Oberflächenrauhigkeit erzeugt, welche im Bereich zwischen 20 und 70 Mikrometer von Rt inklusive ist.

13. Verfahren nach einem der Ansprüche 1 bis 12, **dadurch gekennzeichnet, dass** die Beizlösung eines oder eine Mischung enthält aus: eine Mischung aus Ammonium-Bifluorid und Salpetersäure; Ammonium-Fluorid-Ammonium-Bifluorid in Säuremischungen; auf Fluorwasserstoffsäure basierende Mischungen; Natriumflorid in Säuremischungen; Ammonium-Bifluorid und Ammonium-Acetat in Wasser; auf Chlorwasserstoffsäure basierende Mischungen; eine Mischung aus Schwefel- und Chlorwasserstoffsäure; und zumindest eines aus Fluoridsalz, zumindest eine Säure und Wasser.

14. Verfahren nach einem der Ansprüche 1 bis 13, **dadurch gekennzeichnet, dass** der Schritt des Beizens des Implantats mit aufgerauter Oberfläche durch ein Eintauchen des Implantats in einer Mischung aus 50 Gramm eines Pulvers aus Ammonium-Bifluorid, 400 ml einer 65%-Salpetersäure und Wasser vorgenommen wird, um eine Lösung aus einem Liter zu erstellen.

15. Verfahren nach Anspruch 14, **dadurch gekennzeichnet, dass** das Implantat in der Mischung aus Ammonium-Bifluorid, Salpetersäure und Wasser zwischen 15 und 30 Sekunden bei Raumtemperatur eingetaucht wird.

16. Verfahren nach Anspruch 14 oder 15, **dadurch gekennzeichnet, dass** die Mischung aus Ammonium-Bifluorid, Salpetersäure und Wasser bei einer Temperatur zwischen 20 °C und 25 °C, insbesondere 22 +/- 2 °C aufrecht erhalten wird.

17. Verfahren nach einem der Ansprüche 1 bis 16, **dadurch gekennzeichnet, dass** die mechanische Wirkung die Verwendung von Ultraschall, wenn in einem flüssigen Medium platziert, enthält.

18. Verfahren nach einem der Ansprüche 1 bis 17, **dadurch gekennzeichnet, dass** die mechanische Wirkung das Bestrahlen des Implantats mit einem im Wesentlichen Nicht-Schleifpartikel-Medium enthält.

19. Verfahren nach Anspruch 18, **dadurch gekennzeichnet, dass** das im Wesentlichen Nicht-Schleifpartikel-Medium in einem gasförmigen oder flüssigen Strahlmedium angetrieben wird.

20. Verfahren nach Anspruch 18 oder 19, **dadurch gekennzeichnet, dass** die im Wesentlichen Nicht-Schleifpartikel-Medien zumindest eines enthalten aus Trockeneis-Pellets, kristalline Partikel eines wasserlöslichen Materials und Partikel eines bioaktiven Strahlmaterials.

21. Verfahren nach Anspruch 20, **dadurch gekennzeichnet, dass** die Trockeneis-Pellets Kohlendioxid-Schneeflocken enthalten.

22. Verfahren nach Anspruch 20 oder 21, **dadurch gekennzeichnet, dass** die Trockeneis-Pellets einen mittleren Durchmesser von 3 mm haben und in komprimierter Luft bei einem mittleren Druck von 11 bar angetrieben werden.

23. Verfahren nach Anspruch 22, **dadurch gekennzeichnet, dass** die Zufuhrrate von Trockeneis-Pellets in der komprimierten Luft im Wesentlichen im Bereich von 100 kg pro Stunde ist.

24. Verfahren nach einem der Ansprüche 20 bis 23, bei welchem die Bestrahlung mit Trockeneis-Pellets für eine Dauer zwischen 20 Sekunden und 3 Minuten stattfindet.

25. Verfahren nach Anspruch 20, **dadurch gekennzeichnet, dass** die kristallinen Partikel des wasserlöslichen Materials Partikel von zumindest einem enthalten aus Zucker, Natriumchlorid, Natriumsulfat und eine Mischung aus einem jeglichen aus den zuvor genannten Materialien.

26. Verfahren nach Anspruch 20, **dadurch gekennzeichnet, dass** die Partikel des bioaktiven Bestrahlungsmaterials Partikel aus Kalziumphosphat und/oder Kalziumkarbonat enthalten.

27. Verfahren nach einem der Ansprüche 1 bis 26, **dadurch gekennzeichnet, dass** das Knochenimplantat eines enthält aus Titan, Zirkonium, Niobium, Tantal, eine basierend auf einem jeglichen der zuvor genannten Elemente basierende Legierung, ein rostfreier Stahl medizinischer Güte, und eine Kobalt-Chrom-Legierung.

28. Verfahren nach einem der Ansprüche 1 bis 27, bei welchem das Implantat zwischen dem Beizschritt und dem mechanischen Reinigungsschritt einem Spülen in Wasser unterworfen wird, um jegliche Beizlösung-Verunreinigungen zu entfernen.

29. Verfahren nach Anspruch 28, bei welchem das Wasser ein entionisiertes und gefiltertes Wasser ist.

## Revendications

1. Procédé de finition de surface d'un implant osseux, comprenant les étapes consistant à:
dégrossir une surface de l'implant par une projection de particules abrasives ; à
décaper l'implant dégrossi en surface dans une solution de décapage ; et à
nettoyer la surface dégrossie de l'implant par une action mécanique pour détacher de ladite surface les particules projetées décollées,
dans lequel l'étape de décapage comprend une attaque chimique de la surface de l'implant qui sépare ou décolle d'éventuelles particules de projection abrasives partiellement incrustées susceptibles de contaminer la surface de l'implant,
**caractérisé en ce que** :
l'étape de décapage est ajustée de telle sorte que la surface de l'implant conserve une rugosité sensiblement identique à celle produite par la projection de particules abrasives.

2. Procédé selon la revendication 1, dans lequel l'attaque chimique est spécifique du matériau de l'implant et non du matériau qui constitue les particules projetées.

3. Procédé selon l'une quelconque des revendications 1 ou 2, dans lequel l'attaque chimique est isotrope.

4. Procédé selon l'une quelconque des revendications 1 à 3, dans lequel la surface de l'implant est attaquée sur une épaisseur inférieure à 20 µm par l'étape de décapage.

5. Procédé selon l'une quelconque des revendications 1 à 3, dans lequel la surface de l'implant est attaquée sur une épaisseur comprise entre 4 µm et 10 µm par l'étape de décapage.

6. Procédé selon l'une quelconque des revendications 1 à 3, dans lequel la surface de l'implant est attaquée sur une épaisseur comprise entre 2 µm et 4 µm par l'étape de décapage.

7. Procédé selon l'une quelconque des revendications 1 à 3, dans lequel la surface de l'implant est attaquée sur une épaisseur comprise entre 1 µm et 2 µm par l'étape de décapage.

8. Procédé selon l'une quelconque des revendications 1 à 7, **caractérisé en ce que** les particules abrasives utilisées pour dégrossir la surface de l'implant sont des particules de céramique et/ou de métal.

9. Procédé selon la revendication 8, **caractérisé en ce que** les particules abrasives de céramique comprennent au moins soit des particules d'oxyde, soit des particules de nitrure, soit des particules de carbure.

10. Procédé selon l'une quelconque des revendications 1 à 9, **caractérisé en ce que** les particules abrasives sont propulsées dans un milieu de projection gazeux ou liquide.

11. Procédé selon l'une quelconque des revendications 1 à 10, **caractérisé en ce que** l'étape de dégrossissage de la surface de l'implant produit une rugosité de surface allant de 3 à 7 µm de Ra inclus.

12. Procédé selon l'une quelconque des revendications 1 à 11, **caractérisé en ce que** l'étape de dégrossissage de la surface de l'implant produit une rugosité de surface allant de 20 à 70 µm de Rt inclus.

13. Procédé selon l'une quelconque des revendications 1 à 12, **caractérisé en ce que** la solution de décapage comprend l'un ou un mélange des éléments suivants : un mélange de bifluorure d'ammonium et d'acide nitrique ; du fluorure d'ammonium ou du bifluorure d'ammonium dans des mélanges acides ; des mélanges à base d'acide fluorhydrique ; du fluorure de sodium dans des mélanges acides ; du bifluorure d'ammonium et de l'acétate d'ammonium dans de l'eau ; des mélanges à base d'acide chlorhydrique ; un mélange d'acide sulfurique et d'acide chlorhydrique ; et au moins un sel de fluorure, au moins un acide et de l'eau.

14. Procédé selon l'une quelconque des revendications 1 à 13, **caractérisé en ce que** l'étape de décapage de l'implant dégrossi en surface consiste en une immersion de l'implant dans un mélange de 50 g de poudre de bifluorure d'ammonium et de 400 ml d'acide nitrique à 65 % complété d'une quantité d'eau permettant d'amener la solution à 1 litre.

15. Procédé selon la revendication 14, **caractérisé en ce que** l'implant est immergé dans le mélange de bifluorure d'ammonium, d'acide nitrique et d'eau à température ambiante pendant environ 15 à 30 secondes.

16. Procédé selon la revendication 14 ou la revendication 15, **caractérisé en ce que** le mélange de bifluorure d'ammonium, d'acide nitrique et d'eau est maintenu à une température comprise entre 20 °C et 25 °C, en particulier à 22 ± 2 °C.

17. Procédé selon l'une quelconque des revendications 1 à 16, **caractérisé en ce que** l'action mécanique comprend l'utilisation d'ultrasons lorsqu'elle se déroule en milieu liquide.

18. Procédé selon l'une quelconque des revendications 1 à 17, **caractérisé en ce que** l'action mécanique comprend la projection sur l'implant d'une matière particulaire sensiblement non abrasive.

19. Procédé selon la revendication 18, **caractérisé en ce que** les matières particulaires sensiblement non abrasives sont propulsées dans un milieu de projection gazeux ou liquide.

20. Procédé selon la revendication 18 ou la revendication 19, **caractérisé en ce que** les matières particulaires sensiblement non abrasives comprennent au moins soit des pastilles de glace sèche, soit des particules cristallines d'un matériau soluble dans l'eau, soit des particules d'un matériau de projection bioactif.

21. Procédé selon la revendication 20, **caractérisé en ce que** les pastilles de glace sèche comprennent des flocons de neige de dioxyde de carbone.

22. Procédé selon la revendication 20 ou la revendication 21, **caractérisé en ce que** les pastilles de glace sèche ont un diamètre moyen de 3 mm et sont propulsées dans de l'air comprimé à une pression moyenne de 11 bars.

23. Procédé selon la revendication 22, **caractérisé en ce que** le débit d'alimentation en pastilles de glace sèche de l'air comprimé est sensiblement de l'ordre de 100 kilogrammes par heure.

24. Procédé selon l'une quelconque des revendications 20 à 23, dans lequel la projection de pastilles de glace sèche dure de 20 secondes jusqu'à 3 minutes.

25. Procédé selon la revendication 20, **caractérisé en ce que** les particules cristallines de matériau soluble dans l'eau comprennent des particules d'au moins un élément parmi un sucre, du chlorure de sodium, du sulfate de sodium et un mélange de l'un quelconque des matériaux susnommés.

26. Procédé selon la revendication 20, **caractérisé en ce que** les particules de matériau de projection bioactif comprennent des particules de phosphate de calcium et/ou de carbonate de calcium.

27. Procédé selon l'une quelconque des revendications 1 à 26, **caractérisé en ce que** l'implant osseux est constitué soit de titane, soit de zirconium, soit de niobium, soit de tantale, soit d'un alliage à base de l'un quelconque des éléments susnommés, soit d'acier inoxydable de qualité médicale, soit d'un alliage de cobalt et de chrome.

28. Procédé selon l'une quelconque des revendications 1 à 27, dans lequel entre l'étape de décapage et l'étape de nettoyage mécanique, l'implant est soumis à un rinçage à l'eau destiné à le débarrasser de toutes les impuretés de la solution de décapage.

29. Procédé selon la revendication 28, dans lequel l'eau est de l'eau déionisée et filtrée.
